## Europäisches Patentamt

## European Patent Office

## Office européen des brevets

(19)

(11) Veröffentlichungsnummer: **0 244 557**
**A1**

(12) ## EUROPÄISCHE PATENTANMELDUNG

(21) Anmeldenummer: 87100979.1

(22) Anmeldetag: 24.01.87

(51) Int. Cl.⁴: **A 61 K 9/50**, A 61 B 17/22

(30) Priorität: 30.04.86 DE 3614657

(43) Veröffentlichungstag der Anmeldung: **11.11.87**
**Patentblatt 87/46**

(84) Benannte Vertragsstaaten: **CH DE FR IT LI**

(71) Anmelder: **DORNIER MEDIZINTECHNIK GMBH,
Postfach 1128, D-8034 Germering 1 (DE)**

(72) Erfinder: **Schwarze, Werner, Dr., Johanniter Strasse 4,
D-7768 Stockach (DE)**

(74) Vertreter: **Landsmann, Ralf, Dipl.-Ing., Kleeweg 3,
D-7990 Friedrichshafen 1 (DE)**

(54) **Pharmaka enthaltende Lipidvesikel, Verfahren zu ihrer**

(57) Pharmaka die zur Lokaltherapie oder Diagnose verwendet werden und ihre pharmakologische Substanz im menschlichen oder tierischen Körper dort abgeben sollen, wo sie benötigt werden.

EP 0 244 557 A1

ACTORUM AG

DORNIER MEDIZINTECHNIK GMBH    -1-
Postfach 1128
8034 Germering 1

Reg. M 110 EU

Pharmaka enthaltende Lipidvesikel, Verfahren zu ihrer Herstellung und Einbringung in den Körper eines Lebewesens und Freisetzung der in den Lipidvesikeln enthaltende Pharmaka

Die Erfindung betrifft Pharmaka, die zur Lokaltherapie oder Diagnose verwendet werden und ihre pharmakologische Substanz im menschlichen oder tierischen Körper dort abgeben sollen, wo sie benötigt werden.

Aus Forschungsarbeiten auf dem Gebiet der Membranbiophysik sind mehrere Verfahren zur Herstellung von Lipidvesikeln und Liposomen bekannt (z.B. mittels Ultraschall, Dialyseverfahren Gefrier-Tau-Methode, Quellen von Lipidfilmen durch Wasser), deren Durchmesser je nach Lipid, Lipidgemisch und Herstellungsweise zwischen 200 A und 100 µm variieren.

Diese Lipidvesikel oder Liposomen besitzen im Inneren eine wässrige Phase in der das freizusetzende Pharmaka gelöst werden kann. Die Lipidhülle der Vesikel oder Liposomen stellt eine Energiebarriere dar, so daß der Austausch zwischen intravesikulärer und extravesikulärer wässriger Phase stark reduziert ist. Dies bedeutet, daß die Leckrate über die Lipidmembran für das eingeschlossene Pharmaka für Tage im Einzelfall für Wochen vernachlässigbar ist. Die Lipidvesikel oder Liposomen sind für das Pharmaka dicht.

Spritzt man diese Lipidvesikel bzw. Liposomen intravenös verteilen sich diese über den ganzen Körper.

Der Erfindung liegt die Aufgabe zugrunde, Pharmaka berührungsfrei und dosierbar für die lokale Behandlung oder Diagnose freizugeben.

Diese Aufgabe wird erfindungsgemäß durch die Patentansprüche gelöst.

Durch fokussierte Beschallung eines Zielvolumens, welche die Lipidvesikel oder Liposomen vorübergehend öffnet, kann das eingeschlossene Pharmaka in Abhängigkeit von der Anzahl der Stoßwellen dosiert und örtlich definiert freigesetzt werden. Bei ausreichend durchbluteten Organen (z.B. Niere, Leber) werden ständig neue Lipidvesikel oder Liposomen über den Blutkreislauf nachgeliefert, die pharmakabeladen sind.

Depoteffekte der Lipidvesikel oder Liposomen und damit des eingeschlossenen Pharmakas können mit mehreren Methoden erreicht werden.

Die Lipidmembran der Vesikel oder Liposomen kann durch oberflächenaktive Substanzen derart manipuliert werden, daß diese sich gezielt an Strukturen im Zielorgan anlagern. Durch das Öffnen der Vesikel oder Liposomen mittels Stoßwellen kann lokal eine Pharmakadosis erreicht werden.

Die Größe der Lipidvesikel oder Liposomen in Kombination mit einem gezielten Applikationsweg, kann bedingt durch Filtration (Passage zu großer Vesikel/Liposomen nicht möglich) im Blutkreislauf zu lokalen Anreicherungen führen. Das Öffnen dieser angereicherten Lipidvesikel/Liposomen erfolgt mittels Stoßwellen. Für eine Anzahl medizinischer Anwendungen (z.B. Zytostatika, Anäststika) kann eine fraktionierte Freisetzung von therapeutischem oder diagnostischem Vorteil sein. Dies ist durch Beschallung des Zielvolumens in nahezu beliebigen Zeitintervallen möglich.

- 4 -

Die Freisetzung kann unterstützt werden durch

- lokale Hyperthermie

- Röntgenexposition des Zielvolumens

- Injektion von Gasen oder Partikeln ins Zielvolumen.


Die lokale Freisetzung des intravesikulären Volumens von im
Blut verteilten Vesikeln ist beispielsweise bei den folgenden Anwendungen vorteilhaft einsetzbar:


- Zytostatika könnten direkt im oder in der Umgebung des
  Zielorgans freigesetzt werden.


- Anästatika können örtlich begrenzt freigesetzt werden und
  quasi lokal betäuben.


- Blutungsverhindernde Substanzen im Vesikelvolumen können
  bei berührungsfreier Lithotripsie mittels Stosswellen
  gleichzeitig freigesetzt werden.


- Durch Wiederholung von Schüssen in bestimmten Zeitintervallen (einige Minuten) werden bei stark durchbluteten Organen ständig neue Pharmaka freigesetzt.

- 5 -

- Lokal freigesetzte Tracersubstanzen könnten für diagnostische Anreicherungs- oder Flußuntersuchungen benutzt werden.

- Lokale Auflösung von Thrombosen durch Freisetzung von entsprechenden Pharmaka.

- Lokale Hyperthermie durch Freisetzung von Substanzen, die in einer nachfolgenden chemischen Reaktion mit positiver Wärmetönung das betreffende Volumen lokal überhitzen

Die Erfindung wird nachfolgend anhand von Figuren näher beschrieben.

Fig. 1 eine Lipiddoppelschicht
Fig. 2 eine aus einer Doppellipidschicht gebildete Vesikel und
Fig. 3 eine Einrichtung zur Freisetzung von Pharmaka

Der Aufbau biologischer Membranen hat in dem Modell der "fluid-mosaic-membrane" seine heute allgemein akzeptierte Form gefunden.

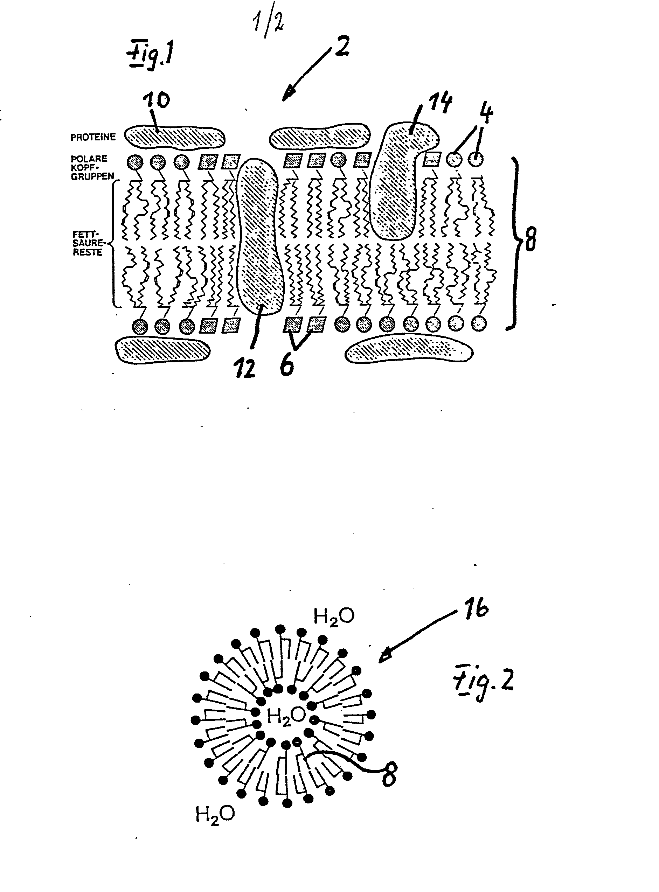

Lipidmoleküle 2 mit hydrophilen Enden 4 (polar) und hydrophoben Enden 6 (unpolar) formen eine Lipiddoppelschicht 8 der Art, daß die hydrophoben Enden einander zugewandt und die

- 6 -

hydrophilen Köpfe die Grenzfläche zur jeweils wässrigen Phase bilden. Die beiden Schichten sind dementsprechen antiparallel orientiert, wie Fig. 1 zeigt.

Das unpolare Membraninnere stellt für geladene Ionen der wässrigen Phase der Umgebung eine hohe Energiebarriere dar, sie sind lipidunlöslich. Eine Ausnahme sind lipidlösliche Substanzen.

Weiterhin wird angenommen, daß die Lipidmembran sich in einem "flüssig-kristallinen" Zustand befindet, der zwar laterale Platzwechselzeiten von $10^{-7}$s zuläßt, transversaler Austausch jedoch nur im Bereich von Stunden oder Tagen möglich ist. Der Widerstand künstlicher Lipidmembranen aus reinem Lipid beträgt etwa $10^{8}\Omega$ cm und ist damit etwa $10^{6}$ mal höher als der von Zellmembranen.

Das Modell der Doppellipidschicht hat unter anderem seine Be-stätigung in elektronenmikroskopischen Aufnahmen gefunden. Der Versuch künstliche Lipiddoppelschichten in kleinen Öffnungen einer hydrophoben Trennwand zu erzeugen, gelang erstmals 1962 und kann zusammen mit der Möglichkeit elektrochemisch aktive Transportproteine in solche planaren Lipiddoppelschichten als weitere, wichtige Unterstützung des vorgeschlagenen Modells angesehen werden. Messungen an solchen Lipidmembranen mit eingebauten Transportsystemen haben erheblich zum Verständnis von Transportprozessen in Membranen beigetragen.

Beim Aufbau von Vesikeln unterscheidet man zwischen einschaligen (aus einer Doppellipidschicht) und mehrschaligen (aus mehreren Doppellipidschichten bestehend) Vesikeln.

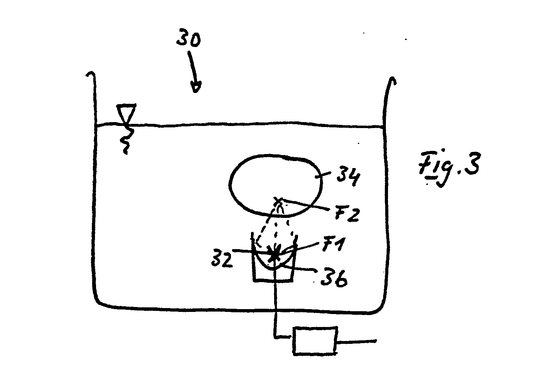

Fig. 2 zeigt das Modell einer Vesikel 16 nach Ultraschallbehandlung, wobei die Vesikel aus einer Doppellipidschicht 8 gebildet ist.

Die Größe einer einschaligen Vesikel variiert zwischen 200-1000 $\overset{o}{A}$ in Abhängigkeit von der Herstellungsweise und den benutzten Lipiden. Erhöhung der Membrandicke (lange Fettsäureketten) führt zu größeren Vesikeldurchmessern. Mehrschalige Vesikel können bis zu einer Größe von max. 100 µm hergestellt werden. Bei einem Durchmesser von 1 µm wird in der Literatur auch von Liposomen gesprochen. Diese sind lichtmikroskopisch erkennbar.

Die variierbaren Parameter sind im Folgenden kurz zusammengefaßt:

- Größe der Vesikel
- Lipidzusammensetzung. Gemische aus Lipiden sind möglich Damit kann die Starrheit (Haltbarkeit) beeinflußt werden.

- Intravesikuläres Medium

- Außenmedium.

Des weiteren ist es möglich Proteine 10, 12, 14 in die Vesikelmembran einzubauen oder anzulagern.

- Vesikel 16 (Fig. 2) werden mit verschiedenen Methoden hergestellt, die auch kombiniert eingesetzt werden können. Die Herstellug erfolgt im allgemeinen

   - im Ultraschallbad

   - mit Hilfe des Dialyseverfahrens

   - mit einer speziellen Gefrier-Tau-Methode.

   - oder quellen von Lipidfilmen

Die genaue maximale Dichte der Vesikel 16 in Suspension ist derzeit nicht bekannt, dürfte aber in der Größenordnung von einem Volumenprozent der Suspension liegen.

Fig. 3 zeigt eine Einrichtung zur Freisetzung von Pharmaka in prinzipieller Darstellung. Innerhalb einer wassergefüllten Wanne 30 befinden sich eine Stoßwellenquelle 32 und ein Patient 34. Die Stoßwellenquelle befindet sich im Ausführungsbeispiel im Brennpunkt F 1 eines Rotationsellipsoids
36. Von der Stoßwellenquelle ausgehende Stoßwellen werden im
Fokus F 2 konzentriert. Dort befindet sich das Organ und die
Lipidvesikeln, aus denen Pharmaka freigesetzt werden sollen.

Zur Erzeugung von Stoßwellen können Funkenentladung, Laser,
elektromechanische Quellen oder piezoelektrische Erzeuger
verwendet werden. Zur Fokussierung können an Stelle eines
Ellipsoiden auch Kugelkalotten oder Linsenfokussierung dienen.

12.12.86
PdL/mr

DORNIER MEDIZINTECHNIK GMBH
POSTFACH 1128
8034 Germering 1


Reg. M 110 EU


Patentansprüche


1. Freisetzung von in Lipidvesikeln enthaltenen Pharmaka, wobei sich die Lipidvesikel in der Blutbahn von Lebewesen befinden, dadurch gekennzeichnet, daß die im Gewebe befindlichen Lipidvesikel berührungsfrei durch das Gewebe hindurch mit Stoßwellen beschallt werden und die in ihnen enthaltenen Pharmaka vollständig oder teilweise freigeben.


2. Freisetzung von in Lipidvesikeln enthaltenen Pharmaka, wobei sich die Lipidvesikel in der Blutbahn von Lebewesen befinden, dadurch gekennzeichnet, daß die im Gewebe befindlichen Lipidvesikel örtlich und zeitlich definiert mit Stoßwellen beschallt werden und dabei Pharmaka freigeben.


- 2 -

3. Freisetzung von in Lipidvesikeln enthaltene Pharmaka, wobei sich die Lipidvesikel in der Blutbahn von Lebewesen befinden nach Anspruch 2, dadurch gekennzeichnet, daß die gewünschte Menge der freizusetzenden Pharmaka mittels einer einmaligen Stoßwellenbehandlung begrenzter Dauer freigesetzt wird.

4. Freisetzung von in Lipidvesikeln enthaltenen Pharmaka, wobei sich die Lipidvesikel in der Blutbahn von Lebewesen befinden nach Ansprüchen 1 bis 3, dadurch gekennzeichnet, daß die gewünschte Menge der freizusetzenden Phamaka mittels wiederholter oder dauernder Stoßwellenbehandlung freigesetzt wird.

5. Freisetzung von in Lipidvesikeln enthaltenen Pharmaka, wobei sich die Lipidvesikel in der Blutbahn von Lebewesen befinden nach Ansprüchen 1 bis 4, dadurch gekennzeichnet, daß die Stoßwellenbehandlung immer dann fortgesetzt wird, wenn sich frisches Blut mit Pharmaka enthaltenden Lipidvesikeln im Gewebe befindet.

6. Freisetzung von in Lipidvesikeln enthaltenen Pharmaka, wobei sich die Lipidvesikel in der Blutbahn von Lebewesen befinden nach Ansprüchen 1 bis 5, dadurch gekennzeichnet, daß die Stoßwellenbehandlung nach einem Zeitplan erfolgt, der sich an Menge, Wirkungsweise und Wirkungsdauer und Fortschritt des Heilerfolgs der freizusetzenden Pharmaka orientiert.

7. Freisetzung von in Lipidvesikeln enthaltenen Pharmaka, wobei sich die Lipidvesikel in der Blutbahn von Lebewesen befinden nach Ansprüchen 1 bis 6, dadurch gekennzeichnet, daß die Intensität der Stoßwellenbehandlung variierbar ist, wobei der Druckgradient geringer ist als die Zerstörungsschwelle lebenden Gewebes.

8. Freisetzung von in Lipidvesikeln enthaltenen Pharmaka, wobei sich die Lipidvesikel in der Blutbahn von Lebewesen befinden nach Ansprüchen 1 bis 7, dadurch gekennzeichnet, daß mindestens eine fokussierende Stoßwellenquelle vorhanden ist und der Brennfleck variabel gewählt werden kann.

9. Freisetzung von in Lipidvesikeln enthaltenen Pharmaka, wobei sich die Lipidvesikel in der Blutbahn von Lebewesen befinden nach Ansprüchen 1 bis 8, dadurch gekennzeichnet, daß mehrere fokussierende Stoßwellenquellen vorhanden sind und die Stoßwellenfront variabel gestaltet werden kann.

10. Lipidvesikel oder Lipidgemische für die berührungsfreie Freisetzung von Pharmaka mittels Stoßwellen, gekennzeichnet durch Lipidmoleküle mit hydrophilen und hydrophoben Enden, die derart eine Lipiddoppelschicht bilden, daß die hydrophoben Enden einander zugewandt und die hydrophilen Köpfe die Grenzfläche zur jeweils wässrigen Phase der Umgebung bilden und sich im Membraninneren Pharmaka befinden.

11. Lipidvesikel oder Lipidgemische für die berührungsfreie Freisetzung von Pharmaka mittels Stoßwellen, bestehend aus Lipidmolekülen mit hydrophilen und hydrophoben Enden, die derart eine Lipiddoppelschicht bilden, daß die hydrophoben Enden einander zugewandt und die hydrophilen Köpfe die Grenzfläche zur jeweils wässrigen Phase der Umgebung bilden und sich im Membraninneren Pharmaka befinden, dadurch gekennzeichnet, daß Fehlstellen in die Vesikelmembran eingebaut sind, die die Freisetzung der Pharmaka bei Stoßwellenbeaufschlagung begünstigen oder erleichtern.

12. Lipidvesikel für die berührungsfreie Freisetzung von Pharmaka mittels Stoßwellen nach Anspruch 11, dadurch gekennzeichnet, daß die Fehlstellen an die Vesikelmembran angelagert sind.

13. Lipidvesikel für die berührungsfreie Freisetzung von Pharmaka mittels Stoßwellen nach Ansprüchen 11 und 12, dadurch gekennzeichnet, daß die Fehlstellen Proteine sind.

14. Lipidvesikel für die berührungsfreie Freisetzung von Pharmaka mittels Stoßwellen nach Ansprüchen 1 bis 13, dadurch gekennzeichnet, daß die Lipidmembran oberflächenaktive Substanzen aufweist, die derart manipuliert sind, daß sich die Lipidvesikel in einem bestimmen Organ oder Organteil des Lebewesens anlagern.

15. Lipidvesikel für die berührungsfreie Freisetzung von Pharmaka mittels Stoßwellen nach Anspruch 14, dadurch gekennzeichnet, daß sich die Lipidvesikel aufgrund ihrer Größe und/oder ihres Applikationswegs in einem Organ oder Organteil anreichern.

16. Unterstützung der berührungsfreien Freisetzung von Pharmaka, die sich in Lipidvesikeln im lebenden Gewebe befinden, gekennzeichnet durch lokale Hyperthermie, Röntgenexposition oder Injektion von Gasen oder Partikeln, die eine Vielfachreflektion der Stoßwellen im Zielvolumen bewirken.

17. Vorrichtung zur Freisetzung von Pharmaka, die sich in Lipidvesikeln im lebenden Gewebe befinden, dadurch gekennzeichnet, daß sich innerhalb einer wassergefüllten Wanne eine rotationselliptische Fokussierungskammer mit Stoßwellenquelle in ihrem Brennpunkt befindet und sich das zu beschallende Gewebe im zweiten Brennpunkt befindet.

18. Vorrichtung nach Anspruch 17, dadurch gekennzeichnet, daß die Stoßwellen elektromagnetisch erzeugt werden und mittels einer Linse oder eines Linsensystems fokussiert werden.

19. Vorrichtung nach Ansprüchen 17 und 18, gekennzeichnet durch einen elastischen Faltenbalg für die Einkopplung der Stoßwelle.

12.12.86
PaL/mr

Fig.1

**PROTEINE**

**POLARE KOPF-GRUPPEN**

**FETT-SÄURE-RESTE**

Fig.2

$H_2O$

$H_2O$

$H_2O$

Fig.3

0244557

Europäisches
Patentamt

**EUROPÄISCHER RECHERCHENBERICHT**

Nummer der Anmeldung

EP 87 10 0979

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| A | BIOLOGICAL ABSTRACT, Band 75, 1983, Zusammenfassungsnr. 50885; J.R. TACKER et al.: "Delivery of anti-tumor drug to bladder cancer by use of phase transition liposome and hypothermia", & J. UROL. (USA) 1982, 127 (6) 1211-1214 | 1-16 | A 61 K 9/50<br>A 61 B 17/22 |
| A | DE-A-2 747 378 (CHOAY S.A.)<br>* Ansprüche 3, 7-11, 18; Seite 7, Zeilen 23-32; Seite 8, Zeilen 1-11 * | 10 | |
| A | EP-A-0 036 277 (THE REGENTS OF THE UNIVERSITY OF CALIFORNIA)<br>* Seite 3, Zeilen 5-30 * | 14 | |
| A | FR-A-2 564 319 (I.N.S.E.R.M. et C.N.R.S.)<br>* Zusammenfassung; Seite 1, Zeilen 27-32; Seite 2, Zeilen 1-15; Ansprüche 1-5 * | 14-15 | RECHERCHIERTE SACHGEBIETE (Int. Cl.4)<br><br>A 61 K 9/00<br>A 61 B 17/00 |
| A | DE-A-2 718 847 (HAÜSLER et al.)<br>* Anspruch 1, Abbildungen 1-4 * | 17 | |
| A | EP-A-0 131 653 (N.V. OPTISCHE INDUSTRIE)<br>* Zusammenfassung; Abbildung 1; Ansprüche 1, 2 * | 18 | |

--- -/-

Der vorliegende Recherchenbericht wurde für alle Patentansprüche erstellt.

| Recherchenort<br>BERLIN | Abschlußdatum der Recherche<br>01-07-1987 | Prüfer<br>AVEDIKIAN P.F. |
|---|---|---|

0244557

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | DE-A-3 444 421 (DORNIER SYSTEM GMBH) * Anspruch 1 * ----- | 19 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| BERLIN | 01-07-1987 | AVEDIKIAN P.F. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82